# EUROPEAN PATENT SPECIFICATION

(11) **EP 2 457 450 B1**
(45) Date of publication and mention of the grant of the patent: **11.01.2017**
(21) Application number: 11382367.8
(22) Date of filing: 28.11.2011
(51) Int. Cl.: A23K 10/30, A23K 10/37, A23K 20/163, A23K 50/10, A23K 50/75

(54) **Edible composition with stevia rebaudiana**
Essbare Zusammensetzung mit Stevia Rebaudiana
Composition commestible à base de stevia rebaudiana

(30) Priority: 29.11.2010 EP 10382320
(43) Date of publication of application: 30.05.2012
(73) Proprietor: Biolittletec, S.L., 08010 Barcelona (ES)
(72) Inventor: Sanchez-Lafuente Mariol, Luis, 08034 BARCELONA (ES); Cuné Castellana, Jordi, 08191 RUBÍ (ES)
(74) Representative: ZBM Patents ApS

(56) References cited:
- EP-A1- 0 868 917
- EP-A1- 1 106 076
- JP-A- 2005 124 536
- RU-A- 2007 146 863
- RU-C2- 2 376 863
- DATABASE WPI Week 198726 Thomson Scientific, London, GB; AN 1987-180537 XP002161281, & JP 62 108790 A (DOEN F) 20 May 1987 (1987-05-20)
- DATABASE WPI Week 200170 Thomson Scientific, London, GB; AN 2001-609312 XP002621870, & JP 2001 192342 A (TA STEVIA KK) 17 July 2001 (2001-07-17)
- DATABASE FSTA [Online] INTERNATIONAL FOOD INFORMATION SERVICE (IFIS), FRANkFURT-MAIN, DE; 2007, PONOMAREV A N ET AL: "Feeding phytosupplements for improving milk quality.", XP002667998, Database accession no. FS-2007-12-Pe2891 & PONOMAREV, SEMENOV, SHEREMETOVA: "Feeding phytosupplements for improving milk quality", MOLOCHANAYA PROMYSHLENNOST, no. No. 7, 2007, page 27, MOLOCHNAYA PROMYSHLENNOST 2007 VORONEZHSKII GOSUDARSTVENNYI AGRARNYI UNIV. IM. K. D. GLINKI, VORONEZH, RUSSIA
- 'Steviol glycoside content in different organs of Stevia rebaudiana and its dynamics during ontogeny' 01 January 2003, XP055223241

## Description

The present invention aims to a composition for feed, namely for feeding animals. The invention also relates to several uses of the composition which are in the benefit of the animals itself and of the final consumers of these animals or their related products.

### BACKGROUND ART

It is widely known that egg yolk colour dependents on the diet of the hen, so then, if the diet contains the yellow or orange plant pigments known as xanthophylls (compounds from the carotenoid group), then they are deposited in the yolk, and colour it. Lutein is the most abundant pigment in egg yolk. A colourless diet can produce an almost colourless yolk. Yolk colour is for example enhanced if the diet includes products such as yellow corn and marigold petals. In the United States, the use of artificial colour additives is forbidden, thus the feeding pattern of the poultry is critical in order to obtain the desired yolk colour.

Moreover, the colour and brightness of the egg yolk are important quality attributes that allow determining if the egg is in good condition to be eaten. Nonetheless, each region of the world prefers different yolk colours, thus making the poultry keepers to have in mind what the final consumers are in order to feed the animals accordingly.

It is then usual to incorporate red and yellow pigments (some from natural sources, others from synthetic processes) in the bird feed. For several decades the yellow-orange colour has been provided by the synthetic cantaxanthin. Also, and as exposed in the patent document US3539686 it is possible to obtain a wide range of tones from yellow to red using xantophylls in addition to other pigments such as cantaxanthin, beta-apo-8-carotenoic acid, extracts of paprika and red peppers.

Yellow xantophylls may be obtained from yellow corn, marigold meal, and alfalfa, meanwhile the red pigment is provided by the synthetic cantaxanthin (Hoffman-La Roche, BASF) disclosed above. However, the use of some synthetic pigments is limited in several countries. Natural red pigments may be obtained from pepper, and yellow synthetic pigments from citranaxanthin (BASF) or ethyl ester of beta-apo-8-carotenoic acid (Hoffman-La Roche).

Another way to adjust the egg yolk colouration is by means of a feed supplemented with Spirulina, a blue-green alga in the shape of a spiral coil, living both in sea and fresh water. Spirulina is the common name for human and animal food produced primarily from two species of cyanobacteria: Arthrospira platensis, and Arthrospira maxima. Though referred to as 'algae' because they are aquatic organisms capable of photosynthesis, cyanobacteria are not related to any of the various eukaryotic algae. Spirulina contains many pigments, including chlorophyll-a, xanthophyll, beta-carotene, echinenone, myxoxanthophyll, zeaxanthin, canthaxanthin, diatoxanthin, 3'-hydroxyechinenone, beta-cryptoxanthin and oscillaxanthin, plus the phycobiliproteins c-phycocyanin and allophycocyanin.

The key to pigmentation of egg yolks with Spirulina is the presence of carotenoids in the same. It is then critical to use a supply containing the highest concentrations to maintain colouration. However, different worldwide produced Spirulina gives rise to different colouring properties, with some colouring batches poor in carotenoids if poor growth conditions are present.

Due to the rising demand of attractive "healthy-seeming" food, and also keeping in mind the health of the animals, there is a need of alternative pigmentation sources that comply with the regulatory provisions of the different demanding countries.

### SUMMARY OF THE INVENTION

Inventors have found that by incorporating a specific mixture of the leaves and the stem of the Stevia rebaudiana plant in the fodders of hens, the egg yolk colouration is improved and the egg shell is hardened. Thus, inventors have provided an edible composition which is useful as egg yolk colouration agent as well as egg shell hardening agent.

To the best of the inventor's knowledge the use of Stevia rebaudiana has neither been suggested as pigmentation agent, nor as egg yolk colouration agent. Stevia rebaudiana is most widely known as sweetener agent, in the form of extract as well as in natural form (the plant itself).

This edible composition is safe for the animals and allows the regulation of the brightness and colour of the egg yolk by means of natural ingredients, which may be incorporated in the common diet of the animals.

Accordingly, an aspect of the present invention is the provision of an edible composition having an amount of steviol glycosides comprised between 150 parts per million (ppm) and 3000 ppm, the composition comprising a mixture of dried solids (or solid dry mixture) that is a mixture of the leaves and the stem of the Stevia rebaudiana plant, the leaves and the stem being in a ratio comprised between 25:75 and 35:65 w/w %
, wherein the mixture of dried solids in relation with the total weight of the edible composition is comprised between 0.025% - 0.5% by weight in the final edible composition.

In other words, the edible composition comprises, together with any edible acceptable ingredient, an amount of a mixture of dried solids (or solid dry mixture) that is a mixture of the leaves and the stem of the Stevia rebaudiana plant, the leaves and the stem being in a ratio comprised between 25:75 and 35:65 w/w %, said amount giving raise to a final concentration of steviol glycosides comprised between 150 parts per million (ppm) and 3000 ppm with respect to the total weight of the edible composition.

Selection of the edible acceptable ingredient and the most appropriate methods for formulation in view of the particular purpose of the composition is within the scope of ordinary persons skilled in the art of food technology.

The term "edible acceptable ingredient" as used herein pertains to compounds, materials, compositions, and/or dosage forms which are suitable for use in food preparations, such as vitamins, proteins, fibre, carbohydrates, fat, etc., which are widely employed in fodders, nutritional supplements, liquid preparations, etc. Each ingredient must also be "acceptable" in the sense of being compatible with the other ingredients of the formulation.

In the sense of the invention an "amount of steviol glycosides" means that mixtures of the compounds of formula (I) are included in the edible composition, that is, a mixture of stevioside, rebaudiosides A, B, C, D, E and F, dulcoside A, steviolbioside, and rubusoside. Particular proportions of all of them will vary in function of the part of the plant employed. It is to be understood that not all the steviol glycosides have to be present in the mixture.

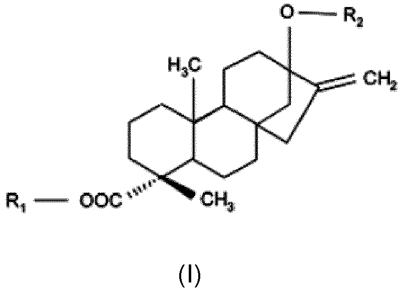

Stevia rebaudiana Bertoni is one of the 240 species of the genus Stevia in the family of Asteraceae. Stevia rebaudiana Bertoni is a plant from South America tropical origin extensively cultivated and marketed in Paraguay. For centuries, the Guaraní tribes of Paraguay, Bolivia and Brazil used Stevia, which they called "ka'a he'ê" ("sweet herb"), as a sweetener in yerba mate and medicinal teas for treating heartburn and other aliments. It is a plant with other multiple uses, and it has been widely used for diabetic people. Also popular traditions attribute to Stevia rebaudiana properties such as anti-aging agent, antiarthritic and anti-arthrosic product, allergy alleviation agent, anti-hypertensor, and as good for preserving a good pancreas state associated to type II diabetes.

Nowadays, the molecular spectrum of Stevia rebaudiana extracts is still deeply studied. Anyway, it is widely known that stevioside and rebaudosides are the active principles responsible of the sweetener effect. The obtention of the sweetener agents of the plant is generally performed by a first infusion of the plant, by secondly filtering the impurities and then performing an extraction with specific solvents to extract the active principles. This process implies that most of the ingredients of the plant with antibiotic properties are lost or decomposed.

Most of the properties attributed to Stevia rebaudiana are due to the presence of steviol glycosides of formula (I). They result from the diterpene known as Steviol (formula II), which is the aglycone of stevia's sweet glycosides.

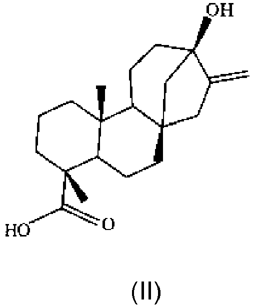

Following Table A lists the identified steviol glycosides. They are constructed by replacing steviol's carboxyl hydrogen atom (R₁) with glucose to form an ester, and replacing the hydrogen of the hydroxyl (R₂) with combinations of glucose and rhamnose. Steviol is also named (4α)-13-hydroxykaur-16-en-18-oic acid with CAS Registry Number 471-80-7. The two primary steviol derivative compounds, stevioside and rebaudioside A, use only glucose: Stevioside, the compound of formula (III) or (4-α)-13-[(2-O-β-D-Glucopyranosyl-β-D-glucopyranosyl)oxy]kaur-16-en-18-oic acid β-D-glucopyranosyl (CAS Registry Number 57817-89-7), has two linked glucose molecules at the hydroxyl site, whereas rebaudioside A has three.

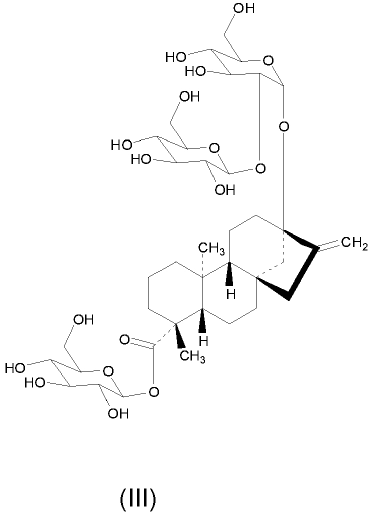

**Table A: Chemical Structures of various steviol glycosides**

| Diterpene glycoside | R₁^{a} | R₂^{a} |
|---|---|---|
| Steviolbioside (V) | H | β-Glc-β-Glc(2-1) |
| Rubusoside (VI) | β-Glc | β-Glc |
| Stevioside (III) | β-Glc | β-Glc-β-Glc(2-1) |
| Rebaudioside A (IV) | β-Glc-β-Glc(2-1) | 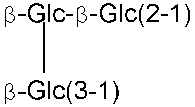 |
| Rebaudioside B (VII) | H | 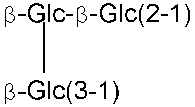 |
| Rebaudioside C (dulcoside B) (VIII) | β-Glc | 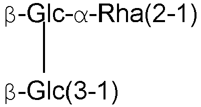 |
| Rebaudioside D(IX) | β-Glc-β-Glc(2-1) | 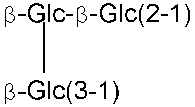 |
| Rebaudioside E (X) | β-Glc-β-Glc(2-1) | 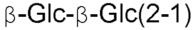 |
| Rebaudioside F (XI) | β-Glc | 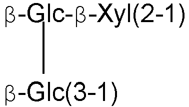 |
| Dulcoside A (XII) | β-Glc | β-Glc-α-Rha(2-1) |

| | | |
|---|---|---|
| ^{a}: Glc is β-D-Glucopyranosyl (glucose); Rha is α-L-rhamnopyranosyl; Xyl is β- D-Xylopyranosyl (xylose); | | |

β-Glc-β-Glc(2-1) means that the radical is a disaccharide of two glucoses with a glycosidic bond established between carbon 2 of the glucose linked to the diterpene structure of formula (I) and carbon 1 of the other glucose (β-D-glucopyranosyl) monomer; 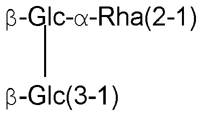 means that the radical is a trisaccharide of two glucose monomers and a rhamnose, wherein a glycosidic bond is established between carbon 3 of the glucose linked to the diterpene structure of formula (I) and carbon 1 of the other glucose (β-D-glucopyranosyl) monomer, and another glycosidic bond is established between carbon 2 of the glucose linked to the diterpene structure of formula (I) and carbon 1 of an α-L-rhamnopyranosyl; 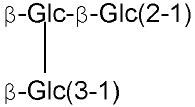 means that the radical is a trisaccharide of three glucose monomers wherein a glycosidic bond is established between carbon 3 of the glucose linked to the diterpene structure of formula (I) and carbon 1 of one glucose (β-D-glucopyranosyl) monomer, and another glycosidic bond is established between carbon 2 of the glucose linked to the diterpene structure of formula (I) and carbon 1 of one glucose (β-D-glucopyranosyl) monomer; 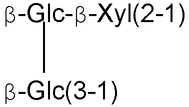 means that the radical is a trisaccharide of two glucose monomers and a xylose (β-D-Xylopyranosyl), wherein a glycosidic bond is established between carbon 3 of the glucose linked to the diterpene structure of formula (I) and carbon 1 of the other glucose (β-D-glucopyranosyl) monomer, and another glycosidic bond is established between carbon 2 of the glucose linked to the diterpene structure of formula (I) and carbon 1 of a β-D-Xylopyranosyl (xylose);
β-Glc-α-Rha(2-1) means that the radical is a disaccharide of a glucose establishing a glycosidic bond between carbon 2 of a α-L-rhamnopyranosyl monomer;
Stevia rebaudiana is widely cultivated around the world (Australia, Canada, India, Mexico, Brazil, Paraguay, Bolivia, etc., although its origin is from South America tropical. For the purpose of the present invention Stevia rebaudiana of different origins and countries may be used. Specially, Stevia rebaudiana from the Amambay mountain chain is used.

A second aspect of the invention is a process for the preparation of an edible composition as defined above and comprising the steps of:
a) drying the plant Stevia rebaudiana at a temperature comprised between 18 °C - 35 °C,
b) separating the stems from the leaves to obtain a dried stem fraction and a dried leaf fraction, and
c) mixing the leaves and the stem fractions of the Stevia rebaudiana plant in a ratio comprised between 25:75 and 35:65 w/w %, to obtain a mixture of dried solids.

As the amount of steviol glycosides must be comprised between 150 ppm and 3000 ppm in the edible composition, the edible composition comprises the appropriate amount of the mixture of dried solids that yields the amount of steviol glycoside previously indicated in the edible composition. The amounts of the mixture of dried solids are comprised between 0.025% - 0.5 % by weight of the mixture of dried solids (25:75 and 35:65 w/w % of leaf:stem) in the final edible composition (e.g. a fodder).

Indeed, the plant may be naturally dried at the environment temperature, or using tumble dryers or heat air driers. When naturally dried, the leaves and stems are extended over a film (for example in a greenhouse) and let the sun heat and dry them.

The edible composition according to the invention is also suitable to be used as an ingredient of several processed food. Thus, a third aspect of the invention is a fodder comprising an effective amount of the edible composition as defined above.

In turn, another aspect relates to the edible composition being itself a processed food, in particular a fodder. In other words, according to another aspect of the invention the edible composition of the invention is a fodder.

The term "effective amount" as used herein, means an amount of an active agent high enough to deliver the desired benefit, but low enough to avoid serious side effects within the scope of medical judgment.

Another aspect of the invention is a method for preparing a fodder as defined above and comprising mixing an animal edible composition in the form of a powder, and an edible composition having an amount of steviol glycosides comprised between 150 ppm and 3000 ppm, the composition in turn, comprising a mixture of dried solids that is a mixture of the leaves and the stems of the Stevia rebaudiana plant, being the leaves and the stems in a ratio comprised between 25:75 and 35:65 w/w %.

Thus, the method for preparing this fodder comprises mixing an animal edible composition in the form of a powder with the edible composition as defined above.

Several particle sizes for the powdered edible composition, to which the edible composition of the invention is added, include from 0.1 mm to 10 mm. This range of particle sizes is also applicable to the mixture of dried solids of leaves and stems of Stevia rebaudiana in the case this mixture of leaves and stems is added also in the form of a powder (by previously grinding the mixture to the desired particle size average). The animal edible composition includes the proteins, vitamins, fat, salts, amino acids, and cellulose usually present in these types of compositions. Variation of the percentages and components may exist depending on the final animal consumer.

The edible composition of the invention is also formulated in such a way that in another aspect of the invention it is used as fodder additive or as nutritional supplement.

As will be illustrated in the Examples below, the edible composition or any fodder containing it, surprisingly affect the resulting colour of the egg yolk, thus providing the art with a new composition that, apart of being good for the poultry due to the properties derived and yet known from Stevia rebaudiana (sweet flavour), serves as a natural pigmentation agent. Therefore, it is also another aspect of the invention the use of the edible composition as defined above as egg yolk colouring agent.

In the sense of the invention an "egg yolk colouring agent" includes any natural or synthetic compound that affect or finally intervenes in the tone of the egg yolk, due to the inherent colouration of the component or due to the transformation of the same into the hen organism.

Apart of this interesting and unexpected effect of the edible composition of the invention, the inventors have found that the edible composition of the invention also unexpectedly improves the resistance of the egg shell. The resistance of the shell is also a critical point for the poultry farmers, especially when the hens become old and the calcification processes involved in the formation of the shell are compromised. If the shell is not resistant enough, when hens lay the eggs they are broken and not useful or able to be marketed anymore.

Thus, another aspect of the invention is the use of the edible composition as egg shell hardening agent.

In document EP 1106076, directed to methods for raising edible animals, the inventors disclosed the use of a pulverized product of a plant tissue of Stevia to be added to feed in an amount of 0.5 % to 7 % by weight. This pulverized product allowed an interesting breaking ratio of eggs of 2.873% in comparison with the controls.

As will be illustrated by way of the following examples, the values of loss by rupture according to the present invention are surprisingly much more interesting. In particular they are under 1.0 %. Without being bound to theory, the inventors postulate that the final amount of steviol glycosides in the feed for animals, together with the proportion of stem and leaf of Stevia rebaudiana, leads to a synergistic effect that allows hardening the egg shell in a great proportion.

As "egg shell hardening agent" is to be understood any compound, composition or combination of ingredients, which at the end intervene in the metabolic pathways involved in the calcification or formation of the egg shell. Examples of prior art agents include the calcium carbonate, and a fine powder derived from mollusc shells, which is rich in the above-referred calcium salt.

The inventors have also found that the specific selected concentration range of steviol glycosides, together with the presence in the edible composition of the selected ratio proportions of stem and leaves of Stevia rebaudiana in the form of a mixture of dried solids, accomplished an improved feed conversion ratios (FCR) and dairy body weight mean gains (DMG), or which is the same, the weight (in grams) gained every day.

Thus, although it is known that Stevia rebaudiana may enhance animal production (see for example RU2376863C2), generally due to a great ingestion of the sweet food and to a greater conversion rate or feed conversion ratio (FCR), the improvement observed in the feed conversion ratios by the use of the edible composition of the present invention, is considered a great contribution to the art. Moreover, although in RU2376863C2 it is disclosed a composition comprising among 18-19 % by weight of an herbal supplement from Stevia rebaudiana, this amount will represent concentrations of steviol glycosides in the composition (fodder) giving raise to compositions with compromised palatability.

Consequently, another aspect of the invention is the use of the edible composition as defined above as animal productivity enhancer.

FCR is the animal weight gain per ingested weight of fodder

The term "animal productivity enhancer" relates to compositions, food supplements or compounds that are given to livestock for efficiently transforming the ingested food to body mass (meat production), or to have great conversion indexes or food conversion ratios, which is a value indicating the Kg of gained weight per Kg of ingested food. An "animal productivity enhancer" may act also by improving the milk production (in terms of quality and/or quantity) or the growing of fur or wool in the animal. Examples of widely used "animal productivity enhancers" are the probiotics such as BioPlus2b, a mixture of Bacillus strains, Short chain Fatty Acids (SCFA) as propionic acid, and copper (Cu). Productivity enhancers are also known as growth promoters, meat enhancers, or animal derived products enhancers (when it relates for instance to milk production, wool, skin, etc.)

It is worth mentioning that the edible composition of the invention uses the Stevia rebaudiana in natural form, that is, as a dry powder or a mixture of dried solids from the leaves and stems of the plant. This is advantageous, since the extraction processes are avoided. Moreover, the presence of all the components of the plant also implies that a percentage of dietetic fibre is incorporated, and what is of special interest: the attributed antibiotic properties of the plant are not lost.

As indicated above, in the case that the mixture of dried solids of the leaves and the stems is used in the form of a powder, a preferred range of particle size goes from 10 µm to 10.0 mm. Most preferred ranges are from 10 µm to 5.0 mm. Alternatively, the mixture of stems and leaves in the indicated proportions may consist in a mixture of solid fragments not reduced to the powdered consistency. It is enough that the mixture may be homogeneously distributed into a fodder in order to assure the animals will consume it while eating the other components of the fodder.

The inventors have also found that Stevia rebaudiana included in an edible composition unexpectedly increased the counts of lactic acid bacteria (LAB). Therefore, it is also an aspect of the invention an edible composition as defined above, for use in the prevention and/or treatment of microbiota imbalance in the gastrointestinal tract of the animals.

In another aspect, the invention relates to an edible composition being a nutritional supplement.

Finally, another aspect of the invention is a nutritional supplement comprising the edible composition as disclosed above, together with at least one compound selected from the group consisting of: vitamins, minerals, fibre, fatty acids and amino acids.

### BRIEF DESCRIPTION OF THE DRAWINGS

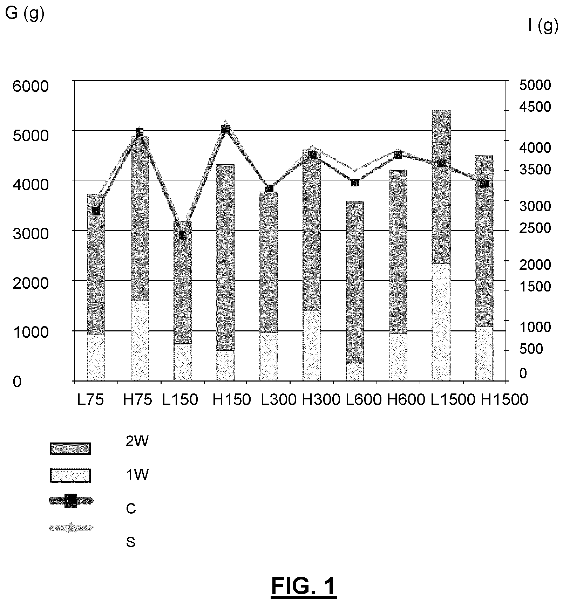

FIG. 1 is a bar and line diagram showing the growing of the animals and the fodder intake of animals, respectively, said animals having access to a fodder comprising a supplement with Stevia (Stevia fodder or S) and a supplement of saccharine (Control fodder or C) during two weeks of treatment. The data from light animals (L) and heavy animals (H) for each steviol glycosides (from Stevia) or saccharine tested concentrations are depicted. The linked lines represent the total intake of fodder comprising Stevia rebaudiana.

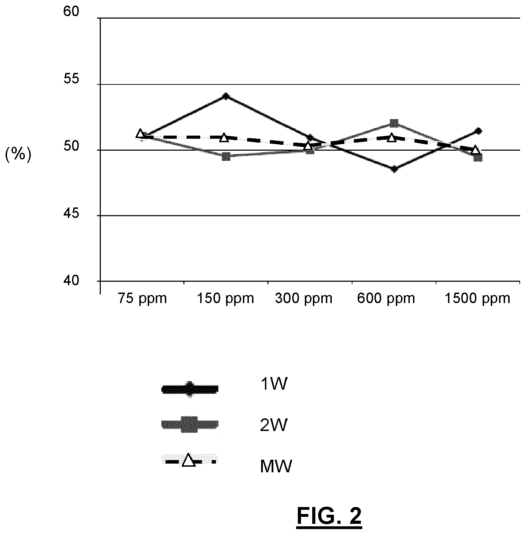

FIG. 2 is a graphic illustrating the percentage of preference studied in piglets for a fodder supplemented with a component of Stevia rebaudiana plant in respect of the control (supplemented with the same amount of saccharine). The ppm indicate the concentration in parts per million of steviol glycosides added in the different tested groups for two weeks (2W). Also the data of the first week (W) are shown, as well as, the mean (MW) of both weeks.

### DETAILED DESCRIPTION OF THE INVENTION

The edible composition of the invention having an amount of steviol glycosides comprised between 150 ppm and 3000 ppm, and a mixture of dried solids that is a mixture of the leaves and the stems of the Stevia rebaudiana plant, the leaves and the stem being in a ratio comprised between 25:75 and 35:65 w/w %, is preferably obtainable by a process comprising the steps of:
a) drying the plant Stevia rebaudiana at a temperature comprised between 18 °C-35 °C,
b) separating the stems from the leaves to obtain a dried stem fraction and a dried leaf fraction, and
c) mixing the leaves and the stem fractions of the Stevia rebaudiana plant in a ratio comprised between 25:75 and 35:65 w/w %, to obtain a mixture of dried solids.

The edible composition includes the amount of this mixture of leaves and stems fractions that give raise to the amounts of steviol glycosides comprised between 150 ppm and 3000 ppm.

Abbreviation w/w % means ratio of percentage by weight.

The previous edible composition is obtainable by the process defined above, further comprising adding the mixture of dried solids from step c) to other components of the composition in such an amount that the edible composition has a final amount of steviol glycosides comprised between 150 ppm and 3000 ppm.

In a preferred embodiment the process further comprises grinding the leaves and the stems of step c) to obtain a powder.

This edible composition has been tested in different experimental assays. From all these assays it is derivable that a preferred embodiment of the invention is an edible composition having an amount of steviol glycosides comprised between 150 parts per million (ppm) and 3000 ppm, the composition comprising a mixture of dried solids (or solid dry mixture) that is a mixture of the leaves and the stem of the Stevia rebaudiana plant, the leaves and the stem being in a ratio comprised between 25:75 and 35:65 w/w %, wherein the mixture of dried solids in relation with the total weight of the edible composition is comprised between 0.025% - 0.5 % by weight in the final edible composition.

Specially preferred is an amount comprised between 0.05 % - 0.5 %, also preferred 0.05 % -0.4 %. An amount of 0.056 % by weight of the mixture of dried solids (25:75 and 35:65 w/w % of leaf:stem) in the final edible composition is most preferred. The Examples illustrates a particular embodiment where 0.056 % of the mixture of dried solids gives raise to 300 ppm of steviol glycosides in the final fodder or edible composition. They also illustrates another particular embodiment where the mixture of dried solids is 0.5 % by weight in the final edible composition. 0.5 % gives raise to 3000 ppm of steviol glycosides in the final fodder or edible composition.

Another preferred embodiment is an edible composition wherein the ratio between leaves and stem of Stevia rebaudiana is 30:70 w/w %.

In another preferred embodiment, the steviol glycosides in the edible composition are in a final concentration comprised between 150 ppm and 1500 ppm, being mostly preferred a final concentration of 300 ppm.

Also in a preferred embodiment the edible composition comprises a mixture of rebaudioside A, stevioside, rebaudioside F, rebaudioside C, dulcoside A, an isolated non-identified compound with molecular weight of 804 g/mol; MW804, rubusoside and rebaudioside B.

The edible composition of the invention may be formulated as a powder or a liquid (i.e. a liquid infusion of the powder or a solution of the powder) to be used as food additive, or in a nutritional supplement, or in a fodder. When formulated as a food additive it may be added to any previously processed feed or liquid, such as milk, vitamin-enriched water, fruit juices, and even be mixed with drug formulations. As above indicated, the edible composition may also be itself a processed food, in particular a fodder or a nutritional supplement.

Thus, also in a preferred embodiment the edible composition according to the invention is a fodder.

In another preferred embodiment, the edible composition comprises the mixture of leaves and stems of Stevia rebaudiana from Paraguay.

The nutritional supplements comprising the edible composition of the invention include all those essential ingredients useful for providing the nutrients, such as vitamins, minerals, fibre, fatty acids, or amino acids, that may be missing or may not be consumed in sufficient quantity in a diet. The edible composition does not prejudice with the supply of all other components.

If, on the other hand, the fodder is the mixture containing the edible composition of the invention, it includes common ingredients of these kind of compositions, such as hay, straw, silage, compressed and pelleted feeds, oils and mixed rations, molasses and also sprouted grains and legumes.

When the edible composition is mixed in a fodder for hens the egg production is bettered in the sense that even old hens produce resistant egg shells, minimizing thus the lost of the production, and moreover, the egg yolk has a good brilliance and colour, namely of yellowish predominant tones. The edible composition including steviol glycosides provided by a component of Stevia rebaudiana plant (stem and leaves), is moreover beneficial for the poultry and animals in general, since it provides them with an interesting amount of digestive fibre. Moreover, the egg yolk has a great brightness, thus demonstrating the good health of the hens and derived eggs. This fact finally allows improving the confidence of the final consumer in respect of the healthy of these hens and of the high quality process to obtain the eggs.

Thus, it is well documented by means of the examples below that Stevia rebaudiana may be used as egg yolk colouring agent and as egg shell hardening agent. For all these egg-related uses, Stevia rebaudiana is preferably used in natural form and the entire plant is employed. This means that the leaves and the stems of the plant give the final interesting results. Thus, it is considered also part of the invention the use of Stevia rebaudiana as egg yolk colouration agent and as egg shell hardening agent. In a preferred embodiment the Stevia rebaudiana is previously reduced to a dry powder; however it may be used in other processed forms, such as simply as collected or previously dried and cut up in order to be eaten by the poultry (namely egg producer hens). Moreover, a wide range of leaf:stem ratios are usable. Indeed, the ratio of the entire plant is also included.

Moreover and as it will be illustrated by way of examples, in the case of animal production the ingestion of the edible composition of the invention provides with several advantages. First of all, the animals eat a great amount of food and the feed conversion ratios (FCR) are also greater. This aspect is of special interest in the field of animal productivity. But moreover, the critical phase and wherein the edible composition of the invention is crucial, is from the weaning phase of the mammals to the fattening phase. This is so, since when the mammals (piglets, kids, rabbits, etc.) are taken away from their mothers, a big stress is impaired to them. The same applies to poultry (chicken, duck, goose, etc.), that get stress when they are stabled.

Thus, the use of the edible composition of the invention as animal productivity enhancer is considered part of the invention. In a preferred embodiment the animal productivity enhancement consist of increasing meat production. This meat is of great quality and provided by means of natural source.

In another preferred embodiment, the animal productivity enhancement consists of increasing milk production.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1. Effect of the composition on the colouring of yolk eggs.

A fodder commonly used for egg producer hens was supplemented by mixing the fodder listed below with the edible composition of the invention incorporating 300 ppm of steviol glycosides, and a powder (1.0 mm to 5.0 mm of particle size) of 30:70 leaves:stem ratio of Stevia rebaudiana from Paraguay (Amambay mountain chain)

The fodder and the water were given ad libitum to 18 hens producing eggs (from Country breed hens). 9 hens received the fodder supplemented with steviol glycosides from Stevia rebaudiana; the control group received only the fodder. The hens were housed in groups of 3 hens. The fodder intake of each group was approximately of 1.0 Kg.

Fodder composition (in percentage by weight): Maize (50 %); toasted soy (18.54 %); wheat (14.50 %); calcium carbonate (9.54%); toasted soy beans (2.42 %); maize gluten (2.00 %); animal fat (1.00 %); calcium biphosphate (0.87 %); sodium chloride (0.51 %), sodium bicarbonate (0.10 %); and vitamin additives (1.03 %)

The employed fodder was a complete fodder for hens in productivity phase (from 2 months to day of sacrifice).

Eggs samples of hens at 1, 3 and 4 months were used for the egg yolk colour determination (9 replicates).

The egg yolks were removed from the egg white and individually homogenized with an agitator. The colour was measured with a Hunter Lab Colorimeter miniScan XTE (Hunter Associates laboratory INC, Reston, Virginia, USA). The following coordinates were measured: CIELAB, L*, a* and b* with an illuminator D65 and a standard observer of 10°.

CIE L*a*b* (CIELAB) is the most complete colour space specified by the International Commission on Illumination (French Commission internationale de l'éclairage, hence its CIE initialism). It describes all the colours visible to the human eye and was created to serve as a device independent model to be used as a reference.

The three coordinates of CIELAB represent the lightness of the colour (L* = 0 yields black and L* = 100 indicates diffuse white; specular white may be higher), its position between red/magenta and green (a*, negative values indicate green while positive values indicate magenta) and its position between yellow and blue (b*, negative values indicate blue and positive values indicate yellow).

Following Table 1 shows the mean of the nine replicates analyzed from egg yolks collected from hens fed with the fodder comprising the edible composition of the invention (sample A), and the mean of the nine control replicates, to which no supplement of the edible composition of the invention was added in the fodder (sample B).
Table 1. Colour of egg yolks

**Table 1. Colour of egg yolks**

| Coordinates (parameters) | A | B |
|---|---|---|
| L* | 62.74 ± 0.62 | 52.69 ± 3.34 |
| a* | 25.79 ± 0.86 | 26.28 ± 1.94 |
| b* | 72.96 ± 1.98 | 64.41 ± 4.21 |

From the data illustrated in Table 1 it is deduced that the egg yolk of hens with a fodder containing the edible composition of the invention have a greater brightness and more intensive yellowish tones than the egg yolks of the controls. This brightness is indicative of the healthy state of the hens.

It is to be noted that the effect on the coloration of the egg yolk is achieved without prejudicing the egg shell resistance to break. Neither the weight of the egg is compromised, in the sense that it does not vary or slightly has higher values.

### Example 2A. Effect on egg shell rigidity. Comparative study of the resistance to break of eggs.

The aim of this assay was to compare the resistance to break of different eggs. This parameter is an indication of the hardness of the egg shell.

Following the same scheme and conditions of Example 1, nine hens were fed with a fodder containing 300 ppm of steviol glycosides from Stevia rebaudiana (powder with particle size from 1.0 mm to 5.0 mm) of 30:70 w/w% leaves:stem ratio of Stevia rebaudiana from Paraguay (Amambay mountain chain) and nine hens only with the fodder.

The eggs were compressed at 5 mm/s according to the methodology disclosed in Voisley and Hunt et al., "Effect of Compression Speed on the Behaviour of Eggshells", J. agric. Engng Res.-1969, Vol. 14(1), pp. 40-46. It was determined if the eggs could challenge the 200 mm/min, as diclosed in Ketelaerea et al., " Measuring the eggshell strength of 6 different genetic strains of laying hens: techniques and comparisons.", Br Poult Sci. - 2002, Vol. 43(2), pp. 238-44.

A texturometer that compressed the egg between two parallel surfaces in the equatorial sense to a distance of 10 % of the initial egg diameter was used. 10 replicates of the eggs from hens supplemented with the edible composition of the invention (sample B) and from the controls (sample A) were analysed.

Following Table 2, show the diameter (in mm) and "force to break" (in newtons), and the distance to break (in mm) of the analyzed eggs.

**Table 2.**

| | Mean A | Mean B | t-value | p | Std.Dev A | Std.Dev B | F-ratio | p |
|---|---|---|---|---|---|---|---|---|
| D | 43.7 | 44.2 | -0.723 | 0.479 | 1.186 | 2.011 | 2.865 | 0.133 |
| FB | 47.9 | 51.7 | -0.862 | 0.400 | 11.16 | 8.015 | 1.937 | 0.339 |
| DB | 0.421 | 0.397 | 0.771 | 0.451 | 0.077 | 0.0634 | 1.473 | 0.573 |
| S | 496 | 509 | -0.686 | 0.501 | 44.84 | 37.17 | 1.455 | 0.585 |

| | | | | | | | | |
|---|---|---|---|---|---|---|---|---|
| D: equatorial diameter of eggs; FB: force to break; DB: Distance to break; S: slope; Std. Dev: Standard deviation. | | | | | | | | |

For "force to break" it has to be understood as the force that has to be applied to detect a rupture in the shell. The "distance to break" are the millimetres that have been reduced from the original equatorial diameter before detecting the rupture. The slope is a parameter indicating the shell rigidity. The slope is calculated from the relation between the force to break and the distance to break, in such a way the greater the slope the greater the rigidity. Thus, an eggshell more rigid means that the eggshell is harder than others presenting a lower value of S.

Eggs of group B were slightly greater that those of group A, with a more rigid shell and which hold a greater force before to be broken.

### Example 2B. Effect of the edible composition in the hen productivity (mortality, productivity, ingestion and conversion index)

In order to demonstrate the effects of the edible composition according to the invention in hens producing eggs, a new assay with a broad sample of animals was performed.

### 3 groups of hens were studied:

Group 1: 12127 hens of 27.5 weeks old, and receiving together with the habitual fodder (Fodder composition of Example 1) an amount of 300 ppm of steviol glycosides from a mixture of dried solids 70:30 w/w% stem:leaves of Stevia rebaudiana. That is, the amount of mixture of dried solids added to the total of the fodder (edible composition) is that allowing 300 ppm of steviol glycosides in the final fodder.

For the preparation of the fodder for Group 1, to 1000 Kg of the fodder composition of Example 1, 560 grams of a mixture of dried solids 70:30 w/w% stem:leaves of Stevia rebaudiana were added. The mixture of dried solids represented then 0.056 % by weight of the total fodder (edible composition).

Group 2: Control group. 12685 hens of 27.5 weeks old receiving only the fodder without the mixture of dried solids 70:30 w/w% stem:leaves of Stevia rebaudiana.

Group 3: 12122 hens of 27.5 weeks old, and receiving together with the habitual fodder (Fodder composition of Example 1) an amount of 3000 ppm of steviol glycosides from a mixture of dried solids 70:30 w/w% stem:leaves of Stevia rebaudiana. That is, the amount of mixture of dried solids added to the total of the fodder (edible composition) is that allowing 3000 ppm of steviol glycosides.

For the preparation of the fodder of Group 3, to 1000 Kg of the fodder composition of Example 1, 5.0 Kg of a mixture of dried solids 70:30 w/w% stem:leaves of Stevia rebaudiana were added. The mixture of dried solids represented then 0.5 % by weight of the total fodder (edible composition).

Hens were kept in three barns at the same conditions of temperature and humidity. The assay was for one month, and data were collected every day. Elected month was June, when due to the environment climatology (summer conditions) the loss of animals is high. Data from the months prior and subsequent to the assay were also collected.

The parameters that were evaluated included: mortality of the hens, productivity in terms of total number of eggs produced per total number of hens during the month of assay, production of Grade A eggs, ingestion of food, and conversion index (CI) in terms of weight of fodders per dozen eggs. In most of the evaluated parameters best results were obtained with Group 1 (fodder including 300 ppm of steviol glycosides).

Mortality data, measured as number of died hens in respect of the total at time 0 (start of the assay) were the following:
- Group 1: 0.214 %
- Group 2 (Control): 0.505 %
- Group 3: 0.346 %

It is directly derivable from the mortality results that the groups (Group 1 and 3) receiving a supplement of steviol glycosides in the form of adequate amounts of a mixture of dried solids 70:30 stem:leaves of Stevia rebaudiana showed lower mortality in respect of the control group (Group 2).

In addition (data not shown) the mortality was maintained, at least for 1 month after depleting the consumption of fodder supplemented with Stevia in both groups 1 and 3.

Productivity data was also evaluated, measured as the total number of eggs produced per total number of hens during the month of assay. According to the bibliography, the optimal productivity for 27.5 weeks old hens is comprised between 90-95 %.

The data obtained for the month of assay were:
- Group 1: 91.46 %
- Group 2: 86.97 %
- Group 3: 87.73 %

As can be seen from the data above, hens of Group 1 maintained the optimal productivity, while the month before the assay (May) all the Groups were in the range of optimal productivity (data not shown). Group 3 showed a value slightly lower than the required, thus depicting a trend to maintain the productivity.

In addition, Group 1 maintained the optimal productivity the month subsequent to the assay (July), meanwhile the other groups did not have productivities between the optimal 90-85 %, although These data allow concluding that an amount of 300 ppm of steviol glycosides provided in the fodder by means of a mixture of dried solids from Stevia rebaudiana with a proportion 70:30 w/w % of stems and leaves, is of special interest to maintain the productivity during critical year periods such as summer when, due to the high temperatures, hens tend to low its productivity (number of eggs/number of hens).

Assaying the parameter of production of Grade A eggs, the following results were collected:
- Group 1: 321500 eggs (96.74 % from the total of eggs produced by Group 1)
- Group 2: 317580 eggs (96.71 % from the total of eggs produced by Group 2).
- Group 3: 307880 eggs (96.17 % from the total of eggs produced by Group 3)

The percentage of eggs lost by rupture were:
- Group 1: 0.68%
- Group 2: 0.97 %
- Group 3: 0.88 %

These data indicate that Group 1 produced 0.59 % more of Grade A eggs (17816 additional eggs). Moreover, the loss due to rupture was notably lower in Groups 1 and 3 in respect of the control (Group 2). At this respect, it is to be noted that the amount of broken eggs in Group 1 was 29.90 % lower than the amount of broken eggs in Group 2.

U.S. Grade A eggs have characteristics of Grade AA eggs except the whites are "reasonably" firm. This is the quality most often sold in stores. Grade AA eggs have whites that are thick and firm; yolks that are high, round, and practically free from defects; and clean, unbroken shells. Grade AA and Grade A eggs are best for frying and poaching, where appearance is important.

With regard to the ingestion of food (intake) in grams per day (g/day), the results were as follows:
- Group 1: 119.15 g/day
- Group 2: 112.12 g/day
- Group 3: 121.20 g/day

During the month of the assay, Groups 1 and 3 had ingested more fodder than the control (Group 2). Nonetheless, the productivity data and other parameters compensate this major ingestion of food.

During prior and post assay months (May and July), the consumption of fodder was similar in all three groups.

Another evaluated parameter was the conversion index (CI), defined as the weight of fodder consumed per dozen eggs produced. The values observed for the three groups were:
- Group 1: 1.45 Kg of fodder/dozen eggs
- Group 2: 1.44 Kg of fodder/dozen eggs
- Group 3: 1.56 Kg of fodder/dozen eggs

Considering the CI values, it was established that the most efficient group receiving the Stevia rebaudiana supplement in the fodder was Group 1, since the CI in relation with Group 3 was similar and the amount of mixture of dried solids to be used in the edible composition (fodder) was lower in order to give raise to a lower concentration of steviol glycosides.

This greater efficiency is moreover corroborated with the values of the productivity data (Group 1 = 91.46 % and Group 3 = 87.73 %).

Nonetheless, a fodder comprising 3000 ppm of steviol glycosides still continues to be of interest considering the results of all the parameters as a whole.

Although data are not included, during the month of the assay it was also evaluated the effect on egg shell rigidity (as in Example 2A), and the colouring of yolk eggs (as in Example 1).

Regarding egg shell rigidity, both groups receiving together with the fodder and amount of a mixture of dried solids of Stevia rebaudiana 70:30 w/w% stems:leaves, this amount leading to a final concentration in the fodder of 300 ppm or 3000 ppm of steviol glycosides, showed values of force to break higher than those of the control group (Group 2). During the month of the assay a higher force to break was needed to break the eggs of Group 1 and Group 3. This force to break was higher along the time during the assay.

In relation with the colouring of yolk eggs, Group 1 and Group 3 gave raise to eggs with a greater brightness and more intensive yellowish tones of the yolks than the controls. This brightness is indicative of the healthy state of the hens.

All the data retrieved from this Example 2B, in which a great sample size of hens allows obtaining meaningful values, show that the edible composition of the invention reduces the stress of the hens during the productive system, thus avoiding the inhibition of the development or the decrease in the productivity due to this stress.

The use of an adequate amount of the mixture of dried solids of Stevia rebaudiana to give raise to the edible composition (fodder) a concentration of steviol glycosides from 150 ppm to 3000 ppm, said mixture of dried solids consisting in a mixture of the leaves and the stems of the plant, the leaves and the stems being in a ratio comprised between 25:75 and 35:65 w/w %, preferably 30:70 w/w%, improves the health of the poultry and reduces the mortality. Ingestion of fodder is moreover incremented but without compromising the conversion index (CI).

### Example 3. A fodder for pigs.

To 490 Kg of a typical piglet fodder (see below in Table 3) 5 Kg of the edible composition of the invention were added. Thus, the edible composition was the 1.00 % by weight of the final supplemented fodder. For the performance of the 5 Kg of edible composition, 3.5 Kg of Stevia stem previously dried and reduced to powder, and 1.5 Kg of Stevia leaves also dried and reduced to powder were mixed and added to the fodder. The edible composition comprised then 0.7 % by weight of stems and 0.3 % by weight of leaves, giving raise to 3000 ppm of steviol glycosides. Stevia rebaudiana was from Paraguay (Amambay mountain chain).

In order to determine the steviol glycoside concentration (ppm) in the leaves and stems of Stevia rebaudiana, several HPLC analysis of these parts of the plant were realized. It was finally determined that starting from 10 microliters of sample (from leaves), 5 mg of steviol glycosides could be obtained having the following percentage distribution: rebaudioside A (31.15 %), stevioside (56.22 %), rebaudioside F (1.05 %), rebaudioside C (5.88 %), dulcoside A (2.32 %), isolated non-identified compound with molecular weight of 804 g/mol; MW804 (1.02 %), rubusoside (1.25 %) and rebaudioside B (1.11 %).

HPLC was performed using two columns (C18 Alltech Alltima, 4.6 mm ID x 25 cm long, and 5 micrometers of particle size) placed in a serial distribution. The mobile phase was acetonitrile / water in a ratio 35/65. The detection was done by UV at 210 nm.

The sample was previously treated by grinding the leaves and drying at 105 °C for 2 hours. Extraction of 0.5 g with 30 ml of boiling water for 10 minutes was performed in closed tubes. Then centrifugation, cooling and two further extraction steps with 30 ml of boiling water were realized. Supernatants of each centrifugation and cooling of the extracts were collected together and an aliquot was used for the HPLC analysis.

The same analysis but using 10 microliters from an extract of the stem of the plant gave the following percentage distribution: rebaudioside A (30.32 %), stevioside (54.06 %), rebaudioside F (1.21 %), rebaudioside C (6.94 %), dulcoside A (3.26 %), isolated non-identified compound with molecular weight of 804 g/mol; MW804 (0.68 %), rubusoside (0.78 %) and rebaudioside B (2.75 %).

HPLC analysis of the stems was performed as indicated by the leaves analysis.

Once the steviol glycosides concentration is determined in the leaves and the stems, the amount of mixture of dried solids comprising leaves and stems of Stevia rebaudiana is calculated to provide in the final fodder (edible composition) the desired parts per million (ppm) of these glycosides. Example 4. Assay on suckling piglets. Determination of the leaf fragment and stem fragment proportions of the Stevia rebaudiana plant component to be used in the edible composition.

The objective of this assay was to demonstrate that piglets fed with Stevia rebaudiana showed higher growth and better conversion rates (kg of feed intake/kg gained by animals).

Among the issues of most research value into the work, was to determine what the most appropriate amount of Stevia rebaudiana as well as the ratio of stem and leaf. Therefore, different proportions of stem and leaf were used. It is important to note that the proportion of stevioside and rebaudioside (or steviol glycosides in general) is higher in the leaf than in the stem, being interesting for the feed organoleptic improvement. However, it has been hypothesized the presence of active ingredients with antiseptic activity in the stem. These substances can have an interesting potential in animal growth, thanks to the modulation of intestinal flora. Improving it, for example by increasing the concentration of LAB (lactic acid bacteria) and reducing the presence of other groups and deleterious genera, as coliforms, can lead to a reduction in the concentration of TNF-alpha. This fact is beneficial for body development, since high concentrations of it, may involve development inhibition by physiological stress.

With all these premises, the following experimental treatments were used: T1: Negative control (NC). Basal diet + Dry alfalfa at 2 % (i.e. 10 kg/ 500 Kg. feed).
T2: NC + Stevia stem at 1.8% + Stevia-leaf at 0.2 % (i.e. 9 Kg + 1 Kg / 500 Kg feed)
T3: NC + Stevia stem at 1.4% + Stevia-leaf at 0.6 % (i.e. 7 Kg + 3 Kg / 500 Kg feed)
T4: NC + Stevia stem at 1.0% + Stevia-leaf at 1.0 % (i.e. 5 Kg + 5 Kg / 500 Kg feed)
T5: Positive control. Basal diet + Dry alfalfa at 2 % (i.e. 10 kg/500 Hg. feed) + CuSO₄ 140 ppm (40 g/500 Kg feed).

All the diets were identical in composition and the experimental products were added at the top of the basal diet. Stevia rebaudiana was from Paraguay (Amambay mountain chain).

The piglets were housed in thirty pens of four animals each. The animals were randomly distributed by initial weight into six blocks. Each block therefore consisted of five pens. Within each block, one of the five treatments was randomly assigned to each of the five pens.

The assay was performed for 42 days and the piglets were weighed at the start, at 14 days and at the end of the experiment (42 days). Initial and final body weight, daily weight gain, feed intake and feed conversion ratio were calculated.

Following Table 3 shows the composition in percentage by weight (%) of the basal diet

**Table 3. Composition of the basal diets (%)**

| Ingredients | pre-starter (from 0 to 21 days post-weaning) | starter (from 21 day post-weaning to aprox. 20 Kg of weight) |
|---|---|---|
| Wheat | 25.00 | 14.00 |
| Barley | 20.00 | 27.00 |
| Maize | 15.00 | 19.15 |
| Sweet milk whey | 10.00 | 5.14 |
| Soya 48 meal | 8.20 | 19.62 |
| Extruded soybeans | 7.25 | - |
| Wheat midlings | 4.00 | 5.73 |
| Potato protein concentrate | 2.62 | - |
| Spray dried porcine plasma | 1.25 | 1.25 |
| Lard | 3.50 | 4.71 |
| Dicalcium Phosphate | 1.51 | 1.62 |
| Calcium carbonate | 0.21 | 0.48 |
| Salt | 0.17 | 0.26 |
| L-Lysine-HCl | 0.49 | 0.39 |
| L-Threonine | 0.16 | 0.12 |
| DL-Methionine | 0.19 | 0.14 |
| L-Tryptophan | 0.05 | - |
| Vit Min complex | 0.40 | 0.40 |

In Table 4, the productive parameters of animals between 0-42 days of experiment are indicated:

**Table 4. Productive parameters.**

| | Initial weight (Kg) | Final weight (Kg) | Daily weight gain (g) | Daily feed intake (g) | Feed to gain ratio | gain to feed ratio (or FCR) |
|---|---|---|---|---|---|---|
| T-2 | 6.47 | 20.37 | 331 | 515 | 1.56 | 0.64 |
| T-3 | 6.48 | 21.32 | 353 | 531 | 1.52 | 0.66 |
| T-4 | 6.46 | 20.96 | 345 | 520 | 1.51 | 0.66 |

Feed to gain ratios and gain to feed ratios are parameters indicating the relation between the daily weight gain and the daily feed intake. The feed to gain ratio indicates the consumption of feed in relation to the weight gained (grams or Kg) of the animal. So, a value over 1 indicates that the animal consumption (in grams or kilograms of fodder) is greater than the weight gain. Gain to feed ratio (which is also called the feed conversion ratio-FCR) is the animal weight gain per ingested weight of fodder.

Next Table 5 depicts the body weight and weight gain of the animals that received the experimental compositions containing Stevia rebaudiana.

**Table 5. Body weight and weight gain of animals calculated using individual animals.**

| | Body weight day 14 (Kg) | Body weight day 42 (Kg) | Daily weight gain (0-14 days) (g) | Daily weight gain (15-42 days) (g) | Daily weight gain (0-42 days) (g) |
|---|---|---|---|---|---|
| T2 | 8.90 | 20.26 | 175 | 406 | 329 |
| T3 | 9.16 | 21.09 | 194 | 426 | 349 |
| T4 | 9.13 | 20.94 | 192 | 422 | 345 |

From de data of Tables 4 and 5 it is to be deduced that treatment T2 appeared to have numerically lower feed intake and weight gain than treatments T3 and T4. This is likely to be due to the higher Stevia stem fraction in this treatment. The best results were obtained with the treatment T3, in which the basal diet was supplemented with Stevia rebaudiana component comprising 30 % of the leaf fragment of the plant and 70 % of the stem fragment of the plant. In all treatments T2 to T5 the amount of mixture of dried solids from the plant Stevia rebaudiana (leaves and stems) represented the 2% by weight of the total edible composition prepared to be eaten.

### Example 5. Preference test. Comparative assay.

In this case, a preference test of pigs with feed supplemented with different concentrations of Stevia rebaudiana was carried out.

To this regard, the parts per million of stevioside and rebaudiosides suitable to be present in the edible composition were determined. The ppm values, namely of steviol glycosides in general could be analysed by the HPLC technique applied to a sample of stem and leaf of the plant as above disclosed (Example 3). In this preference test only the leaf fragment was used.

Another object of this test was the performance of a comparative assay between a fodder supplemented with 150 ppm of steviol glycosides and 150 ppm of saccharine. The reason of using saccharine as comparative control derives from the primary mechanism proposed in which the Stevia rebaudiana potentiates growing due to its sweetener effect.

The test was differenced in the following steps:
Weaning: control pigs from 7-8 kg of weight to 10 kg.
Animals: Hibrid males of Pietrian breed and as reproductive females the line half (Large White x Landrace) half Landrace
Number of animals: 120 animals of a unique band (synchronized labor of 10 sow)
Treatment: 5 simultaneously
Days of fattening: 2 weeks

### Experimental station:

The tests were performed on a premise of 12 yards (10 in test and two nurses). The floor of the pen was formed by plastic slat in its entirety. Each yard had two plate-type hoppers (each with control and treatment diets) where the feed was dosed manually, 4 times a day; and each yard had a drinking trough.

### Feed and water:

Before starting any test a comprehensive analysis of the different types of feed was performed to ensure that they corresponded to the expected formula and were in appropriate microbiological conditions. 2.000 kg of feed were produced.

Following Table 6 illustrates the piglet fodder composition.

**Table 6. Fodder composition in percentage % by weight.**

| Ingredient | % |
|---|---|
| corn | 40,00 |
| barley | 19,39 |
| "soycomil" | 12,50 |
| sweet whey | 10,12 |
| enersoy 3600 | 6,25 |
| s reeng 50% | 5,39 |
| wpc35 | 2,73 |
| Ne-20 Lacto/prest | 2,00 |
| dicalcium phosphate | 0,53 |
| L-Lisine solid 99% | 0,42 |
| Sodium chloride | 0,20 |
| methionine powder | 0,17 |
| threonine | 0,13 |
| valine | 0,10 |
| L-tryptophan 98% | 0,06 |

### Experimental design:

Feed distribution: ad libitum according to the test by giving the feed manually Controls to be made:
- measure of piglet's weight by grouping animals of one yard (12 animals) at weaning day (0), and at days 7 and 14 of treatment
- weak feed consumption by hopper (which correspond with animals grouped in one yard)

The animals were weighed on days 0, 7 and 14 altogether for analyzing the average weight. Then, the animals were separated into heavy and light weighing piglets. The same day it was estimated the feed consumption, by separating the control consumption of the Stevia consumption (with the fodder supplemented with Stevia rebaudiana)

At the end of the experiment the percentage of rejected feed containing Stevia was calculated.

Table 7 as follows is the weight evolution (in Kg) of the piglets receiving together with the fodder an amount of leaf-derived steviol glycosides or saccharine selected from 75 ppm (comparative), 150 ppm, 300 ppm, 600 ppm or 1500 ppm.

**Table 7.**

| Steviol glycoside / saccharine concentration (ppm) | Weight (Kg) at day 0 | Weight (Kg) at day 7 | Weight (Kg) at day 14 |
|---|---|---|---|
| 75 (comparative) | 8.15 | 9.14 | 10.99 |
| 150 | 8.17 | 9.89 | 12.43 |
| 300 | 7.88 | 9.84 | 12.33 |
| 600 | 7.89 | 8.78 | 10.43 |
| 1500 | 7.87 | 9.18 | 11.35 |

It is derived that the animals grew good having access to the control (saccharine) and Stevia-supplemented fodder.

In Table 8 there are indicated the grams of fodder with saccharine (control) and with the edible composition of the invention (Stevia), ingested during the assay.

**Table 8. Fodder intake.**

| Steviol glycoside /saccharine concentration (ppm) | Fodder intake (g) at 0-7 days (Stevia) | Fodder intake (g) at 0-7 days (control) | Fodder intake (g) at 7-14 days (Stevia) | Fodder intake (g) at 7-14 days (control) | TOTAL (Stevia) | TOTAL (control) |
|---|---|---|---|---|---|---|
| 75 (comparative) | 1050.00 | 1428.00 | 2884.00 | 2653.00 | 3934.00 | 4081.00 |
| 150 | 1326.50 | 1522.50 | 3202.50 | 2747.50 | 4529.00 | 4270.00 |
| 300 | 1725.50 | 1774.50 | 2754.50 | 2775.50 | 4480.00 | 4550.00 |
| 600 | 1074.50 | 1484.00 | 2628.50 | 2702.00 | 3703.00 | 4186.00 |
| 1500 | 1326.50 | 1697.50 | 2684.50 | 2726.50 | 4011.00 | 4424.00 |

From Tables 7 and 8 it is concluded that the animals consume the same amount of feed containing Stevia than the feed containing saccharin if steviol glycosides are present in a concentration between 150 ppm and 300 ppm. The best indices of production, both growth and feed utilization, are achieved when the steviol glycosides are present in a concentration between 150 ppm and 300 ppm.

It is important to be noted that he amount of steviol glycosides in the final weight of fodder while allow good productivity (weight evolution, which means produced meat), does not compromise the organoleptic properties of the fodder.

It should be of interest to specifically examine the animals when they only have access to feed containing Stevia, in order to analyze the organoleptic properties and a possible better utilization of the food supplied.

### Example 6. Preference test with a fodder comprising the edible composition including a plant component of Stevia rebaudiana in which the leaf:stem proportion is 30:70.

Once performed the assay of Example 5 in order to determine the preference of a fodder supplemented with Stevia rebaudiana, a new assay was done including in the fodder en edible composition with the same steviol glycosides concentrations, said concentrations being provided by a component of the Stevia rebaudiana plant consisting in a 30 % by weight of its leaf and a 70 % by weight of the stem fragment of the plant. This proportion is considered the best as has been illustrated in Example 4.

Using the leaf and stem fragment of Stevia rebaudiana has not only the economical advantage of making good use of the whole plant, but also this represents an additional fibre apportion which is good for the animals.

The test is differenced in the following step:
Weaning period: control of piglets between 7-8 and 10 kg.
Animals: Hibrid males of Pietrian breed and as reproductive females the line half (Large White x Landrace) half Landrace
Number of animals: 120 animals of a unique band (synchronized labor of 10 sow)
Treatment: 5 simultaneously
Days of fattening: 15 days

### Experimental station:

The tests were performed on a premise of 12 yards (10 in test and two nurses). The floor of the pen was formed by plastic slat in its entirety. Each yard had two plate-type hoppers (each with control and treatment diets) where the feed was dosed manually, 4 times a day; and each yard had a drinking trough.

### Feed and water:

Before starting any test (treatment) a comprehensive analysis of the different types of feed was performed to ensure that corresponded to the expected formula and were in appropriate microbiological conditions. 2.000 kg of feed were produced.

The five treatments with Stevia (70:30 w/w % stem:leaves) were:
Treatment 1 - 75 ppm (comparative): 98 grams of stem and 42 grams of leaf
Treatment 2 - 150 ppm: 197 grams of stem and 84 grams of leaf
Treatment 3 - 300 ppm: 394 grams of stem and 168 grams of leaf
Treatment 4 - 600 ppm: 788 grams of stem and 338 grams of leaf
Treatment 5 - 1500 ppm: 1969 grams of stem and 844 grams of leaf

The fodder supplemented with the above mentioned treatments was the same as that indicated in Table 6 of Example 5.

### Experimental design:

Feed distribution: ad libitum according to the test by giving the feed manually

Controls to be made:
- measure of piglet's weight by grouping animals of one yard (12 animals) at weaning day (0), and at days 7 and 14 of treatment
- weak feed consumption by hopper (which correspond with animals grouped in one yard)

The animals were weighed on days 0, 7 and 14 altogether for analyzing the average weight. Then, the animals were separated into heavy and light weighing piglets. The same day it was estimated the feed consumption by separating the control of the fodder the fodder supplemented with Stevia rebaudiana)

At the end of the experiment the percentage of rejected feed containing Stevia was calculated

Table 9 as follows is the weight evolution (in Kg) of the piglets receiving together with the fodder an amount of leaf and stem-derived steviol glycosides or saccharine, selected from 75 ppm (treatment 1), 150 ppm (treatment 2), 300 ppm (treatment 3), 600 ppm (treatment 4) or 1500 ppm (treatment 5).

**Table 9.**

| Steviol glycosides / saccharine concentration (ppm) | Weight (Kg) at day 0 | Weight (Kg) at day 7 | Weight (Kg) at day 14 |
|---|---|---|---|
| 75 (comparative) | 7.19 | 8.45 | 11.49 |
| 150 | 7.48 | 8.14 | 11.21 |
| 300 | 6.85 | 8.03 | 11.03 |
| 600 | 7.35 | 8.00 | 11.24 |
| 1500 | 6.52 | 8.23 | 11.46 |

It is derived that the animals grew good having access to the control (saccharine) and Stevia-supplemented fodder.

Table 10a lists the grams of fodder with saccharine (control) and with the edible composition of the invention (Stevia), ingested during the assay and separated in function of the piglet weight (light-L or heavy-H).

**Table 10a. Fodder intake.**

| Steviol glycosides /saccharine concentration (ppm) | Fodder intake (g) at 0-7 days (Stevia) | Fodder intake (g) at 0-7 days (control) | Fodder intake (g) at 7-14 days (Stevia) | Fodder intake (g) at 7-14 days (control) |
|---|---|---|---|---|
| L 75 (comparative) | 954 | 923 | 2062 | 1892 |
| H 75 | 1492 | 1431 | 2708 | 2708 |
| L150 | 867 | 750 | 1650 | 1667 |
| H 150 | 1300 | 1083 | 3017 | 3100 |
| L 300 | 1100 | 1033 | 2055 | 2164 |
| H 300 | 1323 | 1308 | 2569 | 2446 |
| L 600 | 877 | 985 | 2618 | 2309 |
| H 600 | 1277 | 1277 | 2569 | 2477 |
| L 1500 | 1133 | 1067 | 2400 | 2545 |
| H 1500 | 1138 | 1077 | 2231 | 2200 |

In Table 10b there are indicated the grams of fodder with saccharine (control) and with the edible composition of the invention (Stevia), ingested during the assay. The values are the consumption mean of light animals and heavy animals.

**Table 10b. Mean of fodder intake.**

| Steviol glycosides /saccharine concentration (ppm) | Fodder intake (g) at 0-7 days (Stevia) | Fodder intake (g) at 0-7 days (control) | Fodder intake (g) at 7-14 days (Stevia) | Fodder intake (g) at 7-14 days (control) | TOTAL (Stevia) | TOTAL (control) |
|---|---|---|---|---|---|---|
| 75 (comparative) | 1223.00 | 1177.00 | 2385.00 | 2300.00 | 3608.00 | 3477.00 |
| 150 | 1083.50 | 916.50 | 2333.50 | 2383.50 | 3417.00 | 3300.00 |
| 300 | 1211.50 | 1170.50 | 2312.00 | 2305.00 | 3523.50 | 3475.50 |
| 600 | 1077.00 | 1131.00 | 2593.50 | 2393.00 | 3670.50 | 3524.00 |
| 1500 | 1135.50 | 1072.00 | 2315.50 | 2372.50 | 3451.00 | 3444.50 |

In FIG. 1 it is schematically indicated the fodder intake (I) of the Stevia supplemented fodder, and the growing (G) of the piglets during the two weeks of treatment. For the growing (G) in grams (represented in the right-hand Y-axis), values for first week (1W) are represented by the light part of the bars and values for the second week (2W) are represented by the dark part of the bars. The data from light animals (L) and heavy animals (H) for each Stevia concentration are depicted. The linked lines represent the total intake (I) of fodder (reproducing the values of fodder intake of Table 10a), and the numerical values in grams are represented in the left-hand Y-axis (I); the black line is the control (C), and grey line is the Stevia group (S). Lxx means light animal receiving xx ppm of saccharine or Stevia supplemented fodder (i.e.: L75 means that the animals received 75 ppm of saccharine or Stevia component with the fodder). Hxx means heavy animal receiving xx ppm of saccharine or Stevia supplemented fodder (i.e.: L75 means that the animals received 75 ppm of saccharine or Stevia component with the fodder).

On the other hand, in FIG. 2 the percentage of preference of the fodder containing the component of Stevia rebaudiana in respect of the control is indicated. Black line indicates the values during the first week (1W); dark-grey line indicates the values during the second week (2W); and with the dashed-black line the mean of the two weeks is depicted (MW). The values indicated in the FIG. 2 represent the percentage (%) of any of the fodder with Stevia in respect of the control (saccharine) consumed by the animals. A 50 % means that both fodders were equally eaten. A value over 50 % indicates that the animals consumed the Stevia fodder in a major degree in respect of the control. 75 ppm, 150 ppm, 300 ppm, 600 ppm, and 1500 ppm are the concentrations of steviol glycosides or saccharine in the assayed fodders.

We note that in the global values of the first two weeks for both subgroups (heavy - light), the preference for saccharine supplemented feed is at least equal than for the Stevia supplemented fodder, and in some case even higher.

From the growth and consumption values another fact can be elucidated: the inclusion of stems in the mixtures implies a greater digestibility or utilization of nutrients from the feed. This fact is also supported by the interesting values obtained in the group supplemented with 1500 ppm, since the previous assay using only the leaf fragment of Stevia did not reflect this possibility (Example 5). Again it was put into manifest that both, proportion of leaves and stems in the mixture of dried solids of Stevia rebaudiana, and the final amount of steviol glycosides are important parameters that contribute together and intimately to achieved the desired effect. In other words, both parameters give raise to the desired effect (enhancement of productivity) while not compromising the taste of the final edible composition given to animals (fodder).

Considering these results as a whole, trying to discover a working mechanism of Stevia rebaudiana, apart from its sweetener effect, has become a priority in the inventor's objectives.

Moreover, keeping in mind economical issues, the most interesting supplementation value was that of 300 ppm. This concentration of steviol glycosides will be used in next Example 7 for the determination of the animal productivity enhancement.

### Example 7. Effect of the edible composition in the feed conversion ratio (FCR). Productivity data.

With the aim of testing if the amounts of Stevia rebaudiana (and especially the concentration of 300 ppm of steviol glycosides) were able to enhance the growing ratios during the weaning of the piglets, the following assay was carried out.

The test was differenced in the following steps:
Weaning period: control of piglets between 6-7 and 20 kg
Animals: Hibrid males of Pietrian breed and as reproductive females the line half (Large White x Landrace) half Landrace
Number of animals: 154 animals of a unique band (synchronized labor of 12 sow)
Treatment: 3 simultaneously
Days of fattening: 42 days

### Experimental station:

The tests were performed on a premise of 12 yards. The floor of the pen was formed by plastic slat in its entirety.

### Feed and water:

Before starting any test a comprehensive analysis of the different types of feed was performed to ensure that corresponded to the expected formula and were in appropriate microbiological conditions. 6.000 kg of feed were produced. The addition of Stevia is done with a proportion of leaf: stem in a 30:70 ratio considering steviol glycosides contained in the two presentations of the plant.

Treatment 300 ppm: fodder supplemented with 394 grams of stem and 169 grams of leaf (70:30 w/w % of stems and leaves).

Treatment 3000 ppm: fodder supplemented with 3938 grams of stem and 1688 grams of leaf (70:30 w/w % of stems and leaves).
Control: fodder without supplementation of steviol glycosides from Stevia rebaudiana.

The fodder supplemented with the abovementioned treatments was the same as that indicated in Table 6 of Example 5. Stevia rebaudiana was from Paraguay (Amambay mountain chain).

### Experimental design:

Feed distribution: ad libitum according to the test by giving the feed manually

Controls to be made:
- individual identification of piglets at the weaning day
- measure of piglet's weight by grouping the animals of one yard (13 animals) at weaning day (0), and day 7 and 14, 21, 28, 35, 42 of treatment
- measure of the weight of individual piglets at weaning day (0) and days 21 and 42 according to the feeding guidelines coinciding with feed changes
- weak feed consumption by hopper (which correspond with animals grouped in one yard (13 animals))

Following Table 11 lists the initial weight mean (IW) in grams of the piglets (data from the weighing in group), the dairy mean of weight gain (DMG) in grams from 0 to 21 days, from 21 to 42 days, and the global from 0 to 42 days of assay; and the dairy mean of ingested fodder (DMI) in grams from 0 to 21 days, from 21 to 42 days, and the global from 0 to 42 days of assay. The feed conversion rate (FCR) is also indicated 0 to 21 days, from 21 to 42 days, and the global from 0 to 42 days of assay.

**Table 11. Data from group weighing**

| | IW | DMG 0-21 days | DMI 0-21 days | FCR 0-21 days | DMG 21-42 days | DMI 21-42 days | FCR 21-42 days | DMG 0-42 days | DMI 0-42 days | FCR 0-42 days |
|---|---|---|---|---|---|---|---|---|---|---|
| T3000 | 6.72 | 195 | 279 | 1.434 | 458 | 696 | 1.520 | 327 | 488 | 1.494 |
| T300 | 6.89 | 198 | 294 | 1.485 | 444 | 695 | 1.566 | 321 | 495 | 1.541 |
| Control | 6.85 | 163 | 256 | 1.573 | 422 | 662 | 1.570 | 292 | 459 | 1.571 |

Next Table 12 shows the data from individual weighing (mean values). namely, there are indicated the dairy mean of weight gain (DMG) in grams from 0 to 21 days, from 21 to 42 days, and the global from 0 to 42 days of assay.

**Table 12. Data from individual weighing.**

| | IW | DMG 0-21 days | DMG 21-42 days | DMG 0-42 days |
|---|---|---|---|---|
| T3000 | 6.82 | 199 | 446 | 322 |
| T300 | 6.96 | 204 | 458 | 331 |
| Control | 6.88 | 173 | 415 | 294 |

From Tables 11 and 12 it is concluded that the piglets fed with a fodder supplemented with Stevia rebaudiana have higher consumption than piglets fed with the control diet. In addition, an improvement in feed conversion of animals was also observed. This improvement is better as the inclusion of Stevia rebaudiana is increased.

The consumption observed in piglets fed with the control fodder is 459 grams per day meanwhile in the piglets fed with Stevia the observed consumption is 495 grams per day (300 ppm) and 487 grams per day (3000 ppm). This increased consumption can be seen both in the first phase (0-21 days) and the second phase (21-42 days) of the assay.

Moreover, it is deduced that the improvement in the feed conversion with the inclusion of Stevia is greater as greater the inclusion in the fodder is; i.e. the rate is improved in 50 grams with the treatment with 300 ppm of steviol glycosides and in 80 grams with 3000 ppm of steviol glycosides. This improvement was specially detected in the first phase of the assay, when the piglets are young and the weaning represents a great stress to them.

The increased consumption and improved feed conversion involve that the growth of the piglets is higher when they are fed with feed (fodder) supplemented with Stevia. While the negative control piglets grew 294 grams per day, Stevia supplemented piglets grew 331 grams per day (300 ppm) and 322 grams per day (3000 ppm). This difference in growth is significant for the treatment of 300 ppm (p = 0,035) and also a tendency for the treatment of 3000 ppm (p = 0.089) is observed.

Schematically, the feed supplemented with 300 ppm of steviol glycosides showed higher growth levels compared with the negative control. However, the difference in grams between this feed and the one supplemented with 3000 ppm is not very high (9 grams) and it has been observed that the higher the Stevia rebaudiana amount (in ppms of steviol glycosides), the better the conversion rate is.

In a further assay performed over the same piglets it was detected that the faeces of the animals that ate the fodder with the edible composition according to the invention, that is, those of T300 and T3000, presented a decrease in the coliform bacteria growing number of about one logarithm (decrease of 90 %). On the other hand, a 10-fold increase of the counts of lactic acid bacteria (LAB) was also observed. This is an interesting point, since it implies an improving of the farm healthiness and a reduction of the infection chances. Moreover, this microbiotic pattern allows reducing the TNF-α concentration, thus minimizing the physiological stress. A parallel study with the aim of detecting the fermentative ability of some LAB to ferment Stevia fibres, revealed that Streptococcus salivarius has a great fermentation power to ferment Stevia rebaudiana fibres, namely those from the leaves of the plant. Thus, it has also to be concluded that Stevia rebaudiana is a good prebiotic that improves the action of beneficial bacteria for the intestinal tract of the animals, such as S. salivarius. A prebiotic is any non-digestible food ingredient that stimulates the growth and/or activity of bacteria in the digestive system which are beneficial to the health of the body. Examples of prebiotics include carbohydrates in fibre form. The invention provides evidences that the edible composition of the invention acts as a prebiotic when administered in a fodder. This further effect is another positive feature of the edible composition, which represents a good active in the field of animal-derived products management.

### Example 8. Effect of Stevia rebaudiana mixture in breastfeeding pigs.

In order to test other parameters related with the productivity enhancement, another assay was performed in pregnant and breastfeeding pigs.

Female pigs were studied. Some pigs (n =15) received a common fodder for pigs (Group 0 or control) and test pigs (n = 12; Group 1) received the fodder of the controls supplemented with 300 ppm of steviol glycosides coming from the adequate amount of a mixture of leaves and stems in a proportion by weight of 30% and 70 % (30:70) in relation with the total of the mixture of leaves and stems (added in the form of a solid dry mixture).

For the preparation of the fodder for Group 1, to 1000 Kg of the common fodder composition of the controls, 560 grams of a mixture of dried solids 70:30 w/w% stem:leaves of Stevia rebaudiana were added. The mixture of dried solids represented then the 0.056 % by weight of the total fodder (edible composition).

Group 1 received the fodder at the end of pregnancy and during breastfeeding (lactation).

Direct productive parameters evaluated were: a) variation of the weight of the pigs, and b) litter growing.

Female pigs were weighed after the delivery and when the weaning of the piglets started (28 days old piglets). The control group females maintained the weight of the delivery (232 Kg) when the weaning started and the piglets started to eat a lacto-initiator fodder.

Test group slightly reduced the weight, from a mean of 236 Kg at the moment of the delivery to 233 Kg when the weaning started.

The values of the weight of the females correspond to the mean of the weight of each group.

Besides, the growing of the litter was as follows. Piglets breastfed with female pigs of the groups 0 and 1 increased their weight in 63.40 Kg and 63.05 Kg, respectively. The values of the weight of the piglets refer to the total of the litter.

Indirect productive parameters evaluated were: a) consumption of lacto-initiator fodder by the piglets, and b) milk secretion by female pigs.

The consumption of lacto-initiator fodder in piglets from females in Group 0 was of 210 g/day, meanwhile the piglets from females in Group 1 consumed only 85 g/day.

All these data taken in combination indicate that piglets breastfed from females of Group 1 consumed lower amounts of lacto-initiator fodder at the same time they gained the same weight than the piglets breastfed from females of Group 0. It can then be said that the milk of females in Group 1 was of good quality and preferred by their respective piglets. Indirectly, it was shown than the production of milk by Group 1 was higher than the production of milk of Group 0, since the piglets grew in equal form.

The parameter of milk production was measured indirectly by calculating the gain in the weight of the littermates in relation with the consumed lacto-initiator fodder.

In other words, piglets in Group 1 ate an amount of lacto-initiator fodder which was 60 % lower than the amount eaten by piglets of Group 0.

Allowing the piglets to be fed with a great proportion of breast milk has the advantage of lowering the consumption of lacto-initiator fodders. Lacto-initiator fodders are the edible compositions supplied to the piglets in substitution of the breast milk. These lacto-initiator fodders are expensive, and they represent one of the challengers of the farmers.

It is then interesting, in terms of efficiency and of profitability of the farms, to give to the littermate the lacto-initiator fodder at a later stage. This can be done by feeding the "mothers" with a fodder (edible composition) comprising the supplement of Stevia rebaudiana in the form of dried solids being a mixture of the leaves and the stems of the plant, the leaves and the stems being in a ratio comprised between 25:75 and 35:65 w/w %, and giving an amount of steviol glycosides from 150 ppm to 3000 ppm in respect of the final fodder weight.

### REFERENCES CITED IN THE APPLICATION

- US3539686
- RU2376863C2
- EP 1106076
- Voisley and Hunt et al., "Effect of Compression Speed on the Behaviour of Eggshells", J. agric. Engng Res.-1969, vol. 14(1), pp. 40-46.
- Ketelaerea et al., " Measuring the eggshell strength of 6 different genetic strains of laying hens: techniques and comparisons.", Br Poult Sci. - 2002, Vol. 43(2), pp. 238-44.

## Claims

1. An edible composition having an amount of steviol glycosides comprised between 150 ppm and 3000 ppm, the composition comprising a mixture of dried solids that is a mixture of the leaves and the stems of the *Stevia rebaudiana* plant, the leaves and the stems being in a ratio comprised between 25:75 and 35:65 w/w %, wherein the mixture of dried solids in relation with the total weight of the edible composition is comprised between 0.025% - 0.5% by weight in the final edible composition.

2. The edible composition according to claim 1, wherein the ratio between leaves and stems is 30:70 w/w %.

3. The edible composition according to any of the claims 1-2, wherein steviol glycosides are in a final concentration comprised between 150 ppm and 1500 ppm.

4. The edible composition according to claim 3, wherein steviol glycosides are in a final concentration of 300 ppm.

5. The edible composition according to any of the claims 1-4, which is a fodder.

6. The edible composition according to any of the claims 1-5, wherein the *Stevia rebaudiana* plant is from Paraguay.

7. A process for the preparation of an edible composition as defined in any of the claim 1-6, comprising:
a) drying the plant *Stevia rebaudiana* at a temperature comprised between 18 °C- 35 °C,
b) separating the stems from the leaves to obtain a dried stem fraction and a dried leaf fraction, and
c) mixing the leaves and the stem fractions of the *Stevia rebaudiana* plant in a ratio comprised between 25:75 and 35:65 w/w %, to obtain a mixture of dried solids.

8. The process of claim 7, further comprising grinding the leaves and the stems of step c) to obtain a powder.

9. The process according to any of the claims 7-8, wherein the mixture of dried solids from step c) is added to other components of the composition in such an amount that the edible composition has a final amount of steviol glycosides comprised between 150 ppm and 3000 ppm.

10. Use of an edible composition as defined in any of claims 1-6, as fodder additive or as nutritional supplement.

11. Use of the edible composition as defined in any of claims 1-6, as egg yolk colouring agent.

12. Use of the edible composition as defined in any of claims 1-6, as egg shell hardening agent.

13. Use of the edible composition as defined in any of claims 1-6, as animal productivity enhancer.

14. Use of the edible composition according to claim 13, wherein the animal productivity enhancement consist of increasing meat production.

15. Use of the edible composition according to claim 13, wherein the animal productivity enhancement consist of increasing milk production.

16. Edible composition as defined in any of the claims 1-6, for use in the prevention and/or treatment of microbiota imbalance in the gastrointestinal tract of the animals.

## Patentansprüche

1. Eine essbare Zusammensetzung, die eine Menge von Steviolglycosiden hat, die bei zwischen 150 ppm und 3000 ppm liegt, wobei die Zusammensetzung eine Mischung aus trockenen Feststoffen umfasst, welche eine Mischung aus den Blättern und aus den Stängeln der Pflanze *Stevia rebaudiana* ist, wobei die Blätter und die Stängel in einem Verhältnis von zwischen 25:75 und 35:65 Gew.-% sind, wobei die Mischung aus trockenen Feststoffen bezogen auf das gesamte Gewicht der essbaren Zusammensetzung bei zwischen 0,025 Gew.-% und 0,5 Gew.-% in der essbaren Endzusammensetzung liegt.

2. Die essbare Zusammensetzung nach Anspruch 1, wobei das Verhältnis von Blättern zu Stängeln 30:70 Gew.-% ist.

3. Die essbare Zusammensetzung nach einem der Ansprüche 1-2, wobei die Steviolglycoside in einer Endkonzentration von zwischen 150 ppm und 1500 ppm sind.

4. Die essbare Zusammensetzung nach Anspruch 3, wobei die Steviolglycoside in einer Endkonzentration von 300 ppm sind.

5. Die essbare Zusammensetzung nach einem der Ansprüche 1-4, welche ein Tierfutter ist.

6. Die essbare Zusammensetzung nach einem der Ansprüche 1-5, wobei die Pflanze *Stevia rebaudiana* aus Paraguay ist.

7. Ein Verfahren zur Herstellung von einer essbaren Zusammensetzung wie in einem der Ansprüche 1-6 definiert umfassend:
a) das Trocknen der Pflanze *Stevia rebaudiana* bei einer Temperatur von zwischen 18 °C - 35 °C,
b) das Abtrennen der Stängel von den Blättern, um einen Trockenstängelanteil und einen Trockenblattanteil zu erhalten, und
c) das Mischen der Blatt- und Stängelanteile der Pflanze *Stevia rebaudiana* in einem Verhältnis von zwischen 25:75 und 35:65 Gew.-%, um eine Mischung aus Trockenfeststoffen zu erhalten.

8. Das Verfahren des Anspruchs 7, weiterhin umfassend das Mahlen der Blätter und der Stängel vom Schritt c), um ein Pulver zu erhalten.

9. Das Verfahren nach einem der Ansprüche 7-8, wobei die Mischung aus Trockenfeststoffen vom Schritt c) zu anderen Bestandteilen der Zusammensetzung in einer Menge hinzugefügt wird, bei der die essbare Zusammensetzung einen Endanteil von Steviolglycosiden von zwischen 150 ppm und 3000 ppm hat.

10. Verwendung einer essbaren Zusammensetzung wie in einem der Ansprüche 1-6 definiert, als Tierfutterzusatzstoff oder als Nahrungsergänzungsmittel.

11. Verwendung der essbaren Zusammensetzung wie in einem der Ansprüche 1-6 definiert als Farbstoff für Eidotter.

12. Verwendung der essbaren Zusammensetzung wie in einem der Ansprüche 1-6 definiert als Härtemittel für Eierschalen.

13. Verwendung der essbaren Zusammensetzung wie in einem der Ansprüche 1-6 definiert als Mittel zur Tierproduktivitätssteigerung.

14. Verwendung der essbaren Zusammensetzung nach Anspruch 13, wobei die Tierproduktivitätssteigerung in einer Erhöhung der Fleischproduktion besteht.

15. Verwendung der essbaren Zusammensetzung nach Anspruch 13, wobei die Tierproduktivitätssteigerung in einer Erhöhung der Milchproduktion besteht.

16. Essbare Zusammensetzung wie in einem der Ansprüche 1-6 definiert zur Verwendung bei der Prävention und/oder Behandlung von Unausgeglichenheit der Mikrobiota in dem Magen-Darm-Trakt von Tieren.

## Revendications

1. Une composition comestible ayant une quantité de glycosides de stéviol comprise entre 150 ppm et 3000 ppm, comprenant la composition un mélange de matière solide séchée qui est un mélange des feuilles et des tiges de la plante *Stevia rebaudiana,* étant les feuilles et les tiges dans un rapport compris entre 25:75 et 35:65 % en poids, dans laquelle le mélange de matière solide séchée par rapport au poids total de la composition comestible est compris entre 0,025% et 0,5% en poids de la composition comestible finale.

2. La composition comestible selon la revendication 1, dans laquelle le rapport des feuilles aux tiges est de 30:70 % en poids.

3. La composition comestible selon l'une quelconque des revendications 1-2, dans laquelle les glycosides de stéviol sont dans une concentration finale comprise entre 150 ppm et 1500 ppm.

4. La composition comestible selon la revendication 3, dans laquelle les glycosides de stéviol sont dans une concentration finale de 300 ppm.

5. La composition comestible selon l'une quelconque des revendications 1-4 qui est un fourrage.

6. La composition comestible selon l'une quelconque des revendications 1-5, dans laquelle la plante *Stevia rebaudiana* est du Paraguay.

7. Un procédé pour la préparation d'une composition comestible telle que définie dans l'une quelconque des revendications 1-6, comprenant :
a) sécher la plante *Stevia rebaudiana* à une température comprise entre 18 °C - 35 °C,
b) séparer les tiges des feuilles afin d'obtenir une fraction de tiges sèche et une fraction de feuilles sèche, et
c) mélanger les fractions de feuilles et de tiges de la plante *Stevia rebaudiana* dans un rapport compris entre 25:75 et 35:65 % en poids afin d'obtenir un mélange de matière séchée solide.

8. Le procédé de la revendication 7, comprenant en outre moudre les feuilles et les tiges de l'étape c) afin d'obtenir une poudre.

9. Le procédé selon l'une quelconque des revendications 7-8, dans lequel le mélange de matière séchée solide de l'étape c) est ajouté à d'autres composants de la composition dans une quantité telle que la composition comestible a une quantité finale de glycosides de stéviol comprise entre 150 ppm et 3000 ppm.

10. Utilisation d'une composition comestible telle que définie dans l'une quelconque des revendications 1-6 comme additif de fourrage ou comme complément nutritionnel.

11. Utilisation de la composition comestible telle que définie dans l'une quelconque des revendications 1-6 comme agent colorant de jaune d'oeuf.

12. Utilisation de la composition comestible telle que définie dans l'une quelconque des revendications 1-6 comme agent durcissant de coquille d'oeuf.

13. Utilisation de la composition comestible telle que définie dans l'une quelconque des revendications 1-6 comme agent pour améliorer la productivité animale.

14. Utilisation de la composition comestible selon la revendication 13, dans laquelle l'amélioration de la productivité animale consiste en augmenter la production de viande.

15. Utilisation de la composition comestible selon la revendication 13, dans laquelle l'amélioration de la productivité animale consiste en augmenter la production de lait.

16. Composition comestible telle que définie dans l'une quelconque des revendications 1-6 pour l'utilisation dans la prévention et/ou le traitement de déséquilibres du microbiote dans le tractus gastro-intestinal des animaux.
